(19) Europäisches Patentamt
European Patent Office
Office européen des brevets

(11) Veröffentlichungsnummer: **0 218 647 B1**

# (12) EUROPÄISCHE PATENTSCHRIFT

(45) Veröffentlichungstag der Patentschrift: **17.06.92**

(51) Int. Cl.$^5$: **C07J 3/00**, A61K 31/56, A61K 7/48

(21) Anmeldenummer: **86902325.9**

(22) Anmeldetag: **10.04.86**

(86) Internationale Anmeldenummer:
**PCT/DE86/00156**

(87) Internationale Veröffentlichungsnummer:
**WO 86/06078 (23.10.86 86/23)**

## (54) DERMATICA.

(30) Priorität: **17.04.85 DE 3514272**

(43) Veröffentlichungstag der Anmeldung:
**22.04.87 Patentblatt 87/17**

(45) Bekanntmachung des Hinweises auf die Patenterteilung:
**17.06.92 Patentblatt 92/25**

(84) Benannte Vertragsstaaten:
**BE CH DE FR GB IT LI LU NL SE**

(56) Entgegenhaltungen:
**GB-A- 1 073 309**

**Steroids, Vol. 2, number 2, August 1963, Holden Day, San Francisco (US) Ralph I. Dorfman: "Antiandrogens in a castrated mouse test", see pages 185-193; respectively 185, 190**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **BITTLER, Dieter**
**Bölkauer Pfad 11**
**W-1000 Berlin 27(DE)**
Erfinder: **LAURENT, Henry**
**Glambecker Weg 21**
**W-1000 Berlin 28(DE)**
Erfinder: **NICKISCH, Klaus**
**Alt Lichtenrade 11**
**W-1000 Berlin 49(DE)**
Erfinder: **RACH, Petra**
**Antwerpener Strasse 1**
**W-1000 Berlin 65(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Steroids,Band 4, Nr. 3, September 1963, Holden Day, SanFrancisco, (US) Albert Segaloff etal.; "anti-androgenic activity of 17,17-dimethyl-18-norandrost-13-enes", siehe Seiten 433-433

Journal of the Chemical Society, Perkin Transactions I, 1972, London, (GB) B. Pelc et al.; "The configuration of 1 alpha-methyl-5 alpha-androstan-3-ones", siehe Seiten 1219-1220

Tetrahedron, Vol. 27, number 11, June 1971, Pergamon Press, Oxford (GB) F.B. Hirschmann et al.: "Formolysis of 3 Beta-acetoxy-5 alpha-pregnan-20 alpha yl-p-toluenesulfonate", see pages 2041-2053 and respectively pages 2043, 2046, 2049

## Beschreibung

Die Erfindung betrifft 1α,17β-Dimethy1-18-nor-steroide der allgemeinen Formel I

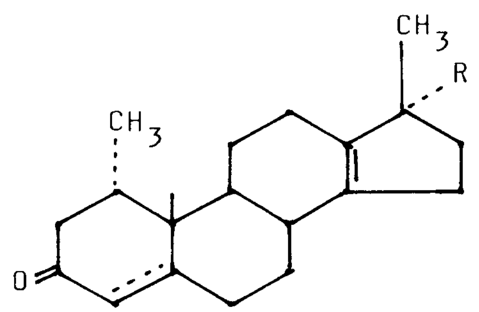

( I ),

worin,
..... eine Einfachbindung oder eine Doppelbindung darstellt und
R eine gegebenenfalls durch eine 2 bis 6 Kohlenstoffatome enthaltende Alkanoyloxygruppe substituierte Alkylgruppe mit maximal 6 Kohlenstoffatomen bedeutet mit Ausnahme des 1α,17,17-Trimethyl-18-nor-5α-androst-13-en-3-on.

Es ist bekannt, daß das 17,17-Dimethyl-18-nor-4,13- androstadien- 3-on und das 17,17-Dimethyl-18-nor-5α-androst-13-en-3-on bei systemischer Applikation schwach antiandrogen wirksam sind (Steroids 2, 1963, 185 und Steroids 4, 1964, 433). Ferner wurden das 17α-Ethyl-17β-methyl-4,13-androstadien-3-on (Chem. Ber. 99,1966,3836 Chem. Ber. 100, 1967, 1169) und Tetrahedron 27, 1971, 2041-2053) und das 1α,17,17-Trimethyl-18-nor-5α-androst-13-en-3-on (J. Chem. Soc. Perkin Transactions I, 1972, 1219-1220) in der Literatur erwähnt, ohne daß etwas über deren pharmakologische Wirksamkeit ausgesagt wurde.

Es wurde nun gefunden, daß die zuvor unbekannten Substanzen der allgemeinen Formel I gemäß Patentanspruch 1 überraschenderweise bei epidermaler Applikation eine starke topische antiandrogene Wirksamkeit besitzen. Zur Bestimmung der antiandrogenen Wirksamkeit wird die Beeinflussung der androgenabhängigen Lipogenese durch die epidermale Applikation der Substanzen wie folgt ermittelt:
Männliche, fertile syrische Goldhamster im Gewicht von etwa 80 bis 100 g werden kastriert und mit täglich 0,1 mg Testosteronpropionat subkutan substituiert. Das rechte Ohr jedes Versuchstieres wird zweimal täglich mit je 0,01 ml einer 1 %igen Lösung der Testsubstanz in Aceton (beziehungsweise bei der Kontrollgruppe nur mit 0,01 ml Lösungsmittel) drei Wochen lang behandelt. Dann werden die Tiere getötet und aus dem behandelten rechten Ohr sowie dem unbehandelten linken Ohr jeweils ein definiertes Gewebeareal von 8 mm Durchmesser ausgestanzt.

Ventrale und dorsale Seite der Ausstanzungen werden entlang des Ohrknorpels voneinander getrennt, unverzüglich in Dulbecco's Modifikation of Eagle's Medium, dem 4 mMol Glutamin und 10 % Kälberserum zugesetzt war und das zur Vermeidung von mikrobieller Kontamination 100 IE/ml Pencillin, 100 µg/ml Streptomycin, 125 µg/ml Kanamycin, 25 IE/ml Nystatin und 10 µg/ml Gentamycin enthält, überführt und eine Stunde lang bei 37 °C inkubiert.

Danach bringt man die Ausstanzungen unter sterilen Bedingungen in frisches Kulturmedium, das 1 µCi/ml $C^{14}$ markiertes Natriumacetat enthält und inkubiert 4 Stunden lang bei 37 °C. Anschließend gibt man die Proben in 2 ml einer Proteolyse-Lösung aus 0,05 Mol Tris-Puffer vom $p_H$ = 7,5, 0,01 mol Dinatriumethylendiamintetraessigsäure, 0,5 % Natriumdodecylsulfat und 0,1 % Proteinase K ( Firma E. Merck A.G. Darmstadt, Bundesrepublik Deutschland), und inkubiert 24 Stunden lang bei 37 °C.

Die so erhaltenen Proben werden einmal mit 5 ml Chloroform und nochmals mit 3 ml Chloroform extrahiert, die vereinigten Chloroformextrakte im Vakuum eingeengt und ihr Gehalt an radiomarkierten Lipiden im Szintillationszähler ermittelt. Die prozentuale Hemmung der Lipogenese der behandelten Kontrollgruppe wird durch Vergleich mit der nur mit Lösungsmittel behandelten Kontrollgruppe berechnet.

Die nachfolgende Tabelle zeigt die in diesen Tests erhaltenen Ergebnisse.

Tabelle

| Nr. | Substanz | Konzentration % | Veränderung der Lipogenese % Rechtes Ohr | Veränderung der Lipogenese % Linkes Ohr |
|---|---|---|---|---|
| 1 | 1α,17,17-Trimethyl-18-nor-4,13-androstadien-3-on | 1,0 | -55 | -12 |
| | | 0,3 | -53 | -16 |
| | | 0,1 | -41 | -11 |
| 2 | 17α-Ethyl-1α,17β-dimethyl-18-nor-5α-androst-13-en-3-on | 1,0 | -48 | -20 |
| | | 0,3 | -40 | - 9 |
| 3 | 1α,17β-Dimethyl-17α-propionyl-18-nor-5α-androst-13-en-3-on | 1,0 | -43 | + 8 |
| 4 | 1α,17β-Dimethyl-17α-(3-propionyloxypropyl)-18-nor-5α-androst-13-en-3-on | 1,0 | -40 | -17 |

Bei subcutaner Applikation zeigen die Verbindungen der allgemeinen Formel I gemäß Patentanspruch 1 nur geringe oder keine systemische antiandrogene Wirksamkeit.

Zur Herstellung von Dermatica können die Verbindungen der allgemeinen Formel I gemäß Patentanspruch 1 mit den üblichen Trägersubstanzen zu Lösungen, Gels, Salben, Pudern oder anderen Präparaten verarbeitet werden. Geeignete Trägersubstanzen sind zum Beispiel Wasser, Ethanol, Propanol, Glycerin,

Methylcellulose, Hydroxypropylcellulose, Carboxypolymethylen u.s.w. Das topisch wirksame Antiandrogen liegt vorzugsweise in einer Konzentration von 0,05 bis 5,0 Gewichtsprozenten, bezogen auf das Gesamtgewicht des Präparates vor. Die Präparate können zur topischen Behandlung von Erkrankungen, wie Akne, Seborrhoe, Alopezie und Hirsutismus verwendet werden.

Die Verbindungen der allgemeinen Formel 1 gemäß Patentanspruch 1, welche als Substituenten $R_2$ beispielsweise eine Methylgruppe, eine Ethylgruppe, eine Propylgruppe, eine Butylgruppe, eine Pentylgruppe, eine Hexylgruppe, eine Isopropylgruppe, eine sec.-Butylgruppe eine Isobutylgruppe oder eine tert.-Butylgruppe - die vorzugsweise terminal durch beispielsweise eine Acetoxygruppe, durch eine Propionyloxygruppe, eine Butyryloxygruppe, eine Dimethylacetoxygruppe, eine Trimethylacetoxygruppe, eine Valeryloxygruppe oder eine Capronyloxygruppe substituiert sein können - tragen können, lassen sich in an sich bekannter Weise herstellen, beispielsweise indem man ein Steroid der allgemeinen Formel II

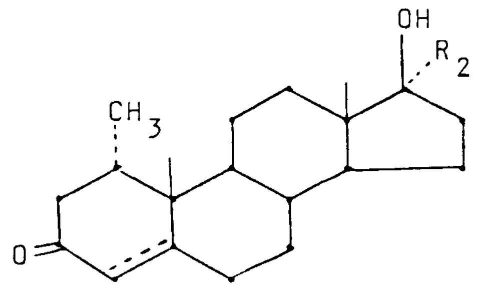

(II),

worin

..... und $R_2$ die im Anspruch 4 genannte Bedeutung besitzt mittels Säuren dehydratisierend umlagert.

Diese Umlagerung kann unter Bedingungen durchgeführt werden die dem Fachmann wohl bekannt sind (Steroids 4, 1964, 433 ff, Chem.Ber. 99, 1966, 3836 ff und Chem.Ber. 100, 1967, 1169) und an Hand typischer Vertreter in den nachfolgenden Ausführungsbeispielen näher erläutert werden.

Beispiel 1

Eine Lösung von 1,5 g 17β-Hydroxy-1α-methyl-17α-propyl-5α-androstan-3-on in 45 ml Essigester und 6 ml Acetanhydrid wird mit 0,06 ml 70 %iger Perchlorsäure versetzt und 15 Minuten bei Raumtemperatur gerührt. Die Reaktionslösung wird mit pyridinhaltigem Eiswasser verdünnt, die Essigesterphase wird abgetrennt und mit Wasser gewaschen. Nach dem Trocknen und Eindampfen werden 1,7 g rohes 3-Acetoxy-1α,17β-dimethyl-17α-propyl-18-nor-5α-androsta-2,13-dien erhalten.

Die Lösung von 1,5 g 3-Acetoxy-1α,17β-dimethyl-17α-propyl-18-nor-5α-androst-2,13-dien in 30 ml Methanol und 15 ml Tetrahydrofuran wird mit 4,5 ml konzentrierter Salzsäure 17 Minuten bei Raumtemperatur gerührt. Die Reaktionslösung wird mit Diethylether verdünnt, mit Wasser neutral gewaschen, getrocknet und eingedampft. Der Rückstand wird an Kieselgel chromatographiert. Ausbeute: 1,04 g 1α,17β-Dimethyl-17α-propyl-18-nor-5α-androst-13-en-3-on vom Schmelzpunkt 88-90° C.

Beispiel 2

Eine Lösung von 6,5 g 17α-Ethy1-3,3-ethylendioxy-1α-methyl-5α-androstan-17β-ol in 65ml 99%iger Ameisensäure wird 15 Minuten bei 100° C gerührt. Die Reaktionslösung wird anschließend in Eiswasser eingerührt, der ausgefallene Niederschlag wird abfiltriert, mit Wasser gewaschen und in Dichlormethan aufgenommen. Nach dem Trocknen und Eindampfen wird der Rückstand aus Methanol umkristallisiert. Es werden 4,05 g 17α-Ethyl-1α,17-dimethy1-18-nor-5α-androst-13-en-3-on vom Schmelzpunkt 122,6° C erhalten.

Beispiel 3

4,5 g 17α-Butyl-17β-hydroxy-1α-methyl-5α-androstan-3-on werden unter den im Beispiel 2 beschriebenen Bedingungen umgesetzt und aufgearbeitet. Nach Chromatographie an Kieselgel erhält man 2,17 g 17α-Butyl-1α,17β-dimethyl-18-nor-5α-androst-13-en-3-on als viskoses Öl.

Beispiel 4

Eine Lösung von 1,0 g 17β-Hydroxy-17α-(3-hydroxypropyl)-1α-methyl-5α-androstan-3-on in 10 ml Propionsäure wird mit 10 ml konzentrierter Salzsäure versetzt und 45 Minuten bei Raumtemperatur gerührt. Die Reaktionslösung wird mit Diethylether verdünnt, mit Wasser und Natriumhydrogencarbonatlösung gewaschen, getrocknet und eingedampft. Der Rückstand wird an Kieselgel chromatographiert. Es werden 780 mg 1α,17β-Dimethyl-17α—(3-propionyloxypropyl)-18-nor-5α-androst-13-en-3-on als viskoses Öl erhalten.

Beispiel 5

1,0 g 17β-Hydroxy-17α-(4-hydroxybutyl)-1α-methyl-5α-androstan-3-on werden unter den im Beispiel 5 beschriebenen Bedingungen umgesetzt und aufgearbeitet. Nach Chromatagraphie an Kieselgel werden 810 mg 1α,17β-Dimethyl-17α-(4-propionyl-oxybutyl)-18-nor-5α-androst-13-en-3-on als zähes Öl erhalten.

Beispiel 6

Eine Lösung von 790 mg 17β-Hydroxy-1α,17α-dimethyl-4-androsten-3-on in 30 ml konzentrierter Ameisensäure wird 10 Minuten auf 100° C erhitzt. Die Reaktionsmischung wird mit Wasser verdünnt und mit Diethylether extrahiert. Der Extrakt wird mit Wasser gewaschen, getrocknet und im Vakuum eingeengt. Der Rückstand wird chromatographiert. Die mit 9 bis 19 % Diethylether-Pentan eluierten Fraktionen enthalten 500 mg, die aus Pentan umkristallisiert, 190 mg 1α,17,17-Trimethyl-18-nor-4,13-androstadien-3-on vom Schmelzpunkt 69,5° C ergeben.
UV: $_{\epsilon\,241}$ = 12 800.

Beispiel 7

1,0 g 17β-Hydroxy-1α-methyl-17α-pentyl-4-androsten-3-on werden unter den im Beispiel 7 beschriebenen Bedingungen umgesetzt. Das Rohprodukt wird chromatographiert. Die mit 22 bis 34 % Diethylether-Pentan eluierten Fraktionen enthalten 1,08 g, die aus Pentan umkristallisiert, 460 mg 1α,17β-Dimethyl-17α-pentyl-18-nor-4,13-androstadien-3-on vom Schmelzpunkt 59,1° C ergeben. UV: $_{\epsilon\,240}$ = 13 500.

Beispiel 8

1,0 g 17α-(4-Acetoxybutyl)-17β-hydroxy-1α-methyl-4-androsten-3-onwerden unter den im Beispiel 7 beschriebenen Bedingungen umgesetzt. Das Rohprodukt wird chromatographiert. Mit 38 bis 44 % Diethylether-Pentan eluiert man 356 mg 17α-(4-Acetoxybutyl)-1α,17β-dimethyl-18-nor-4,13-androstadien-3-on in Form eines viskosen Öls. UV: $_{\epsilon\,241}$ = 12 600.

## Patentansprüche

**1.** 1α,17β-Dimethyl-18-nor-steroide der allgemeinen Formel I

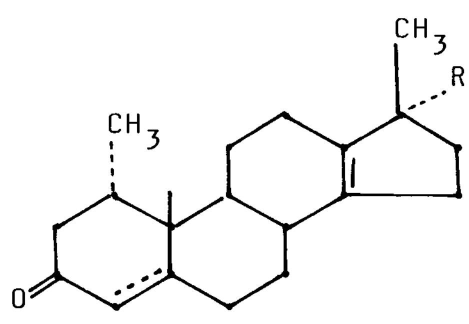

(I),

worin,
..... eine Einfachbindung oder eine Doppelbindung darstellt und
R eine gegebenenfalls durch eine 2 bis 6 Kohlenstoffatome enthaltende Alkanoyloxygruppe substituierte Alkylgruppe mit maximal 6 Kohlenstoffatomen bedeutet mit Ausnahme des 1α,17,17-Trimethyl-18-nor-5α-androst-13-en-3-on.

**2.** 1α,17β-Dimethyl-17α-propyl-18-nor-5α-androst-13-en-3-on-

**3.** 17α-Ethyl-1α,17β-dimethyl-18-nor-5α-androst-13-en-3-on.

**4.** 17α-Butyl-1α,17β-dimethyl-18-nor-5α-androst-13-en-3-on.

**5.** 1α,17β-Dimethyl-17α-(3-propionyloxypropyl)-18-nor-5α-androst-13-en-3-on.

**6.** 1α,17β-Dimethyl-17α-(4-propionyloxybutyl)-18-nor-5α-androst-13-en-3-on.

**7.** 1α,17,17-Trimethyl-18-nor-4,13-androstadien-3-on.

**8.** 1α,17β-Dimethyl-17α-pentyl-18-nor-4,13-androstadien-3-on.

**9.** 17α-(4-Acetoxybutyl)-1α,17β-dimethyl-18-nor-4,13-androstadien-3-on.

**10.** Dermatica, dadurch gekennzeichnet, daß sie als Wirkstoff ein 17β-Methyl-18-nor-steroid gemäß Patentanspruch 2 bis 9 enthalten.

**Claims**

**1.** 1α,17β-dimethyl-18-nor-steroids of the general formula I

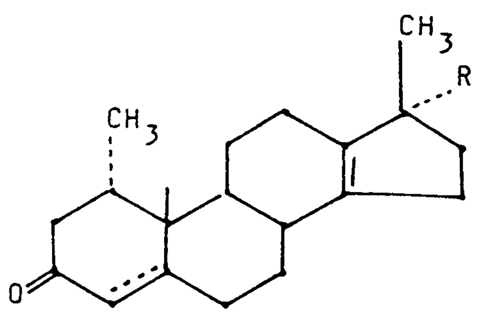

(I)

wherein
..... represents a single bond or a double bond and R represents an alkyl group that has a maximum of 6 carbon atoms and that is optionally substituted by an alkanoyloxy group containing from 2 to 6 carbon atoms, with the exception of 1α,17,17-trimethyl-18-nor-5α-androst-13-en-3-one.

**2.** 1α,17β-dimethyl-17α-propyl-18-nor-5α-androst-13-en-3-one.

**3.** 17α-ethyl-1α,17β-dimethyl-18-nor-5α-androst-13-en-3-one.

**4.** 17α-butyl-1α,17β-dimethyl-18-nor-5α-androst-13-en-3-one.

**5.** 1α,17β-dimethyl-17α-(3-propionyloxypropyl)-18-nor-5α-androst-13-en-3-one.

**6.** 1α,17β-dimethyl-17α-(4-propionyloxybutyl)-18-nor-5α-androst-13-en-3-one.

**7.** 1α,17,17-trimethyl-18-nor-4,13-androstadien-3-one.

**8.** 1α,17β-dimethyl-17α-pentyl-18-nor-4,13-androstadien-3-one.

**9.** 17α-(4-acetoxybutyl)-1α,17β-dimethyl-18-nor-4,13-androstadien-3-one.

10. Dermatics, characterised in that they contain as active ingredient a 17β-methyl-18-nor-steroid according to any one of patent claims 2 to 9.

**Revendications**

1. 1α,17β-Diméthyl-18-nor-stéroïdes répondant à la formule générale I :

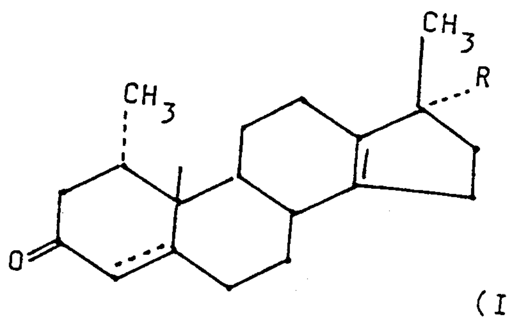

(I)

dans laquelle ... représente une liaison simple ou une liaison double et R représente un radical alkyle qui contient au plus 6 atomes de carbone et qui porte éventuellement un radical alcanoyloxy contenant de 2 à 6 atomes de carbone,
sauf la 1α,17,17-triméthyl-18-nor-5α-androst-13-ène-3-one.

2. 1α,17β-Diméthyl-17α-propyl-18-nor-5α-androst-13-ène-3-one.

3. 17α-Ethyl-1α,17β-diméthyl-18-nor-5α-androst-13-ène-3-one.

4. 17α-Butyl-1α,17β-diméthyl-18-nor-5α-androst-13-ène-3-one.

5. 1α,17β-Diméthyl-17α-(3-propionyloxy-propyl)-18-nor-5α-androst-13-ène-3-one.

6. 1α,17β-Diméthyl-17α-(4-propionyloxy-butyl)-18-nor-5α-androst-13-ène-3-one.

7. 1α,17,17-Triméthyl-18-nor-4,13-androstadiène-3-one.

8. 1α,17β-Diméthyl-17α-pentyl-18-nor-4,13-androstadiène-3-one.

9. 17α-(4-Acétoxy-butyl)-1α,17α-diméthyl-18-nor-4,13-androstadiène-3-one.

10. Produits dermatologiques caractérisés en ce qu'ils contiennent, comme substance active, un 17β-méthyl-18-nor-stéroïde selon l'une quelconque des revendications 2 à 9.